Europäisches Patentamt

European Patent Office

Office européen des brevets

(11) Publication number: **0 105 714**
**B1**

# EUROPEAN PATENT SPECIFICATION

(45) Date of publication of patent specification: **27.07.88**

(21) Application number: **83305834.0**

(22) Date of filing: **28.09.83**

(51) Int. Cl.[4]: **G 01 N 33/577,**
G 01 N 33/68, G 01 N 33/76

(54) **Immunoassay of antigens.**

(30) Priority: **29.09.82 GB 8227826**
**29.09.82 GB 8227828**

(43) Date of publication of application:
**18.04.84 Bulletin 84/16**

(45) Publication of the grant of the patent:
**27.07.88 Bulletin 88/30**

(84) Designated Contracting States:
**BE CH DE FR GB IT LI NL SE**

(56) References cited:
**EP-A-0 048 357**
**EP-A-0 098 590**
**CA-A-1 123 336**
**US-A-4 088 746**
**US-A-4 244 940**
**US-A-4 297 337**
**US-A-4 312 854**

**METHODS IN ENZYMOLOGY, vol. 73,
Immunochemical Techniques, part B G.
GALFRE, C. MILSTEIN "Preparation of
Monoclonal Antibodies: Strategies and
Procedures" pages 3-46**

(73) Proprietor: **Serono Diagnostics Limited**
**21, Woking Business Park Albert Drive**
**Woking Surrey GU21 5JY (GB)**

(72) Inventor: **Forrest, Gordon Coulter**
**8 Chapel Croft**
**Ingatestone Essex (GB)**
Inventor: **Salacinski, Philip Richard Peter**
**144A Portway**
**Stratford London E15 3QW (GB)**
Inventor: **Siddle, Kenneth**
**26 Fairhaven Close**
**Lode Cambridge CB5 9HG (GB)**

(74) Representative: **Woodman, Derek et al**
**FRANK B. DEHN & CO. Imperial House 15-19
Kingsway**
**London WC2B 6UZ (GB)**

(56) References cited:

**NATURE, vol. 256, August 7, 1975 G. KÖHLER,
C. MILSTEIN "Continuous cultures of fused
cells secreting antibody of predefined specifity"
pages 495-497**

Courier Press, Leamington Spa, England.

(58) References cited:

NATURE, vol. 214, May 13, 1967 R. AXEN, J. PORATH, S. ERNBACK "Chemical Coupling of Peptides and Proteins to Polysaccharides by Means of Cyanogen Halides" pages 1302-1304

Scrono MAIA CLONE booklet

HCG MAIA CLONE booklet

Prolactin MAIA CLONE booklet

LH MAIA CLONE booklet

CLIN.Chemistry Vol. 30, No. 9, 1984 pages 1457-1461

## Description

The present invention relates to methods of immunoassay of antigens and to kits for carrying out such methods.

In this specification, the term "antigen" is to be taken to include any substance to which antibodies can be produced, and accordingly includes within its scope haptens, which may have been rendered antigenic for the purpose of producing antibodies.

Immunoassay techniques relay upon the formation of a complex between the antigen being assayed and antibodies which are added as part of the immonoassay procedure. Means are provided whereby the amount of antigen:antibody complex formation is detectable.

There are several known methods of immunoassay employing antibodies which are labelled so as to be analytically identifiable. "Sandwich" or "two-site" techniques involve the formation of a complex between the antigen and two antibody reagents. A convenient method of detecting complex formation between an antigen in a liquid sample and two antibody reagents is to provide that one antibody reagent is labelled and the unlabelled antibody reagent is bound to a solid phase support so that the complex can readily be isolated. Where a radioactive label is employed this technique is known as immunoradiometric assay (IRMA). An example of such an assay is that for thyroid stimulating hormone (TSH) in human serum, in which the serum is mixed with $^{125}$I-labelled sheep anti-TSH antibodies, and with sheep anti-TSH antibodies coupled to a solid phase (e.g. particles). The TSH binds to both antibody reagents and by measuring the amount of label remaining in solution or becoming bound to the solid phase (via TSH), the amount of TSH can be determined.

One difficulty with this type of assay is to produce sufficiently pure labelled antibody. Whilst this can be done, it is a laborious procedure and hence relatively expensive. Recently, this problem has been reduced by the availability of monoclonal antibodies (as opposed to the polyclonal antibodies previously used). Sandwich-type immunoassays involving the use of two monoclonal antibody reagents, a labelled soluble monoclonal antibody and a monoclonal antibody reagent which is bound to a water-insoluble solid phase, are described, for example, in published European patent application No. 0 048 357.

It is a feature of the technique described above that a significant incubation period is normally required to ensure that the reaction goes (so far as is possible) to completion, and this is due at least in part to the fact that the antigen in solution is required to react with antibody bound to a solid phase. With a view to reducing the incubation time, Wolters et al. devised an alternative 2-site assay method, as disclosed in U.S. Patent No. 4,343,896 and outlined below. In the method of Wolters et al., a labelled polyclonal antibody reagent in solution is used together with another soluble polyclonal antibody reagent towards the antigen which is of a different animal species. Complexed antigen-antibody is separated from uncomplexed labelled antibody reagent by employing a third polyclonal antibody reagent coupled to a solid support which is capable of selectively interacting with the unlabelled soluble polyclonal antibody reagent. The advantage of this technique is that the liquid phase reaction (between the antigen, the labelled polyclonal antibody reagent and the other soluble antibody reagent) occurs rapidly, and the reaction with the insolubilized third polyclonal antibody reagent also occurs relatively quickly, thus providing a faster overall time for the assay. The requirement for two antibody reagents directed to different epitopes of the antigen under assay which are also of different animal species has deterred, however, the development of analogous assays employing monoclonal antibody reagents.

We have now devised improved sandwich-type immunoassays for antigens with more than one epitope in a liquid sample wherein a complex is formed in the liquid phase between the antigen under assay and two monoclonal antibody reagents (not necessarily of different animal species), one monoclonal antibody reagent being labelled. The techniques for making monoclonal antibodies are well known (see, for example, Galfre, G. & Milstein, C. (1981) "Preparation of Monoclonal Antibodies: Strategies and Procedures" Methods in Enzymology *73*, 1—46), and will not be further described herein.

In the following portion of this specification, the term "antibody reagent", unless the context dictates otherwise, is to be taken to include reference to intact antibodies and to fragments thereof. The term "fragment", as used herein, unless qualified so as to indicate otherwise, is to be taken to refer to fragments which contain the binding region of the antibody. Such fragments may be Fab-type fragments which are defined as fragments devoid of the Fc portion, e.g. Fab, Fab' and $F(ab')_2$ fragments, or may be so-called "half-molecule" fragments obtained by reductive cleavage of the disulphide bonds connecting the heavy chain components in the intact antibody. The use of monovalent fragments, e.g. Fab, Fab' and half-molecule fragments, is preferred. Methods for preparation of fragments of antibodies are well known in the art and will not be described further herein. The antibody reagents employed in a method according to the present invention may comprise antibodies of any of the various subclasses of immunoglobulins, including in particular IgG and IgM.

The present invention provides a method of immunoassay of an antigen with more than one epitope in a liquid sample which comprises incubating a mixture of:

(a) the liquid sample,

(b) one or more populations of labelled antibodies to the antigen under assay,

(c) a reagent comprising antibodies to the antigen under assay conjugated with a reagent Z, and

(d) a reagent Y capable of binding to reagent Z by non-covalent bonding, but which is not directly bindable to either component (a) or component (b), the said reagent Y being bound to a solid phase support and at least components (b) and (c) comprising monoclonal antibodies;

separating the solids fraction from the liquid fraction, determining the amount of label in one of the said fractions and, therefrom, the amount of antigen present in the sample.

One or more of the antibody reagents may be in the form of fragments. Preferably, the antibodies of components (b) and (c) will be selected so as to be directed against different, roomly-spaced determinants of the antigen.

By the term "non-covalent bonding" as used herein is meant immunological bonding as in an antibody: antigen or antibody: hapten bond or non-immunological bonding such as that between a specific bonding protein and its ligand e.g. in the reaction of protein A and the Fc portion of an antibody or the binding between substances such as avidin and biotin.

The component (b) may be labelled with an analytically indicatable atom or group such as a radioactive atom or group e.g. $^{125}I$, or other methods of labelling known in the art may be used, e.g. fluorimetric labelling or enzyme labelling, which may be direct or indirect, covalent or non-covalent.

The solid support may take the form of finely divided inert particles or beads (e.g. latex particles) and such particles or beads may if desired be magnetic or magnetisable to facilitate the phase separation step. The term "magnetic" as used herein shall be taken to include within its scope ferromagnetic, paramagnetic and superparamagnetic substances. The term "magnetisable" shall be construed accordingly. Suitable magnetic or magnetisable solid supports are described in "Immunoassays for Clinical Chemistry" (Ed. Hunter and Corrie, Churchill Livingstone, Edinburgh (1983)) pp. 147—162; for example, particles of cellulose composite containing $Fe_3O_4$ may be used. In general, any material which facilitates magnetic separation may be employed in the solid support.

Alternatively, the solid support may comprise a generally rod-shaped multi-finned insert. It is important that the surface area of the solid support should be as large as possible. Instead of using particles, beads or insert, the solid phase may comprise a coating on the wall of the reaction vessel (e.g. test tube) or on the wall of a tube through which the reaction mixture passes or it which it is incubated. The phase separation step then consists simply in removing the liquid from contact with the coating. The techniques of binding the reagent Y to suitable supports are well known.

In a preferred embodiment, reagent Z will be a hapten, but antigenic substances such as, for example, proteins, cells, virus particles or membranes may also be employed. Thus, for example, component (c) may comprise monoclonal antibodies to the antigen under assay conjugated with a hapten selected from fluorescein isothiocyanate (FITC), rhodamine isothiocyanate, 2,4-dinitrofluorobenzene, phenyl isothiocyanate and dansyl chloride and reagent Y will then comprise antibodies to the chosen hapten (monoclonal or polyclonal).

Thus, for example, when reagent Z is FITC, reagent Y may conveniently be an anti-FITC polyclonal antibody reagent covalently linked to the solid support. An antiserum to FITC may be readily prepared in conventional manner, for example by immunising sheep with FITC conjugated to keyhole limpet haemocyanin. Coupling of the antiserum to the solid support may be affected for example using the method of Axen *et al* (Nature *214*, 1302—1304 (1967)). Component (c) may for example be prepared by direct reaction of reagent Z with antibody, followed by gel filtration, or component (c) may be prepared by linking reagent Z indirectly to the antibody using bifunctional reagents or spacer groups.

Reagents Y and Z may also, for example, be a specific binding protein and the corresponding ligand, such as for example avidin and biotin which constitute a very rapid, high affinity binding system. The use of such methods of binding offers the great advantage that the reaction can be made very rapid and complete.

It is possible, in certain circumstances, to incorporate reagent Z into component (c) at multiple sites, thus enhancing its reactivity with the solid phase.

Preferably, the antigen is complexed with the soluble antibody reagents prior to the addition of the solid phase component. The labelled and unlabelled antibody reagents may be added in any order, or simultaneously, especially where the antibody reagents are directed against different, roomly-spaced determinants of the antigen. The use of monoclonal reagents for the antibody reagents complexing with the antigen improves the specificity of the process, and also decreases non-specific binding.

In a further aspect, the invention provides kits of reagents for carrying out the assays of the invention. Thus, for example, a kit according to the present invention may comprise (i) particles or beads conjugated to the reagent Y, (ii) labelled component (b), (iii) component (c) and (iv) calibrated reference solutions.

Whilst the method of the invention has very broad applicability, it is particularly useful in assaying the following antigens, namely: hormones, including peptide (e.g. thyroid stimulating hormone (TSH) and human chorionic gonadotrophin (HCG)) or non-peptide (e.g. steroid and thyroid) hormones; proteins (e.g. carcinoembryonic antigen (CEA), alphafetoprotein and immunoglobulins, e.g. IgE); viruses (e.g. heptatis A, hepatitis B and non-A and non-B), allergens, bacteria and toxins and drugs and vitamins with more than one epitope.

In the various aspects of the invention, it is possible to use mixtures of antibodies for the labelled component, e.g, two populations of radioactively-labelled monoclonal antibodies, so as to increase the specific activity of the antibody-antigen-complex (see Example 1) and, as hereinbefore indicated, fragments may be used instead of intact antibodies where this is feasible. The advantages associated with the use of fragments in the methods of the invention instead of intact antibodies are as follows.

A reduction of non-specific binding, i.e. binding between labelled component and a site (e.g. the solid phase support) other than the antigen being assayed is achieved by the use of fragments as the labelled component.

The speed of the assay is increased by the use of fragments which are smaller than intact antibody molecules. The smaller size produces faster reaction with the antigen being assayed.

Monovalent fragments, in contrast to intact antibodies which are bivalent, cannot form aggregates e.g. dimers or more extensive complexes with the antigen. The formation of such aggregates is undesirable in that it may interfere with binding of the complex to the solid phase support.

If, as is generally the case in "sandwich" techniques, the labelled component is used in excess, only one of the valencies of an intact antibody will generally be saturated. This means that one valency is unemployed which reduces the efficiency of the technique.

At high antigen levels, it is possible for two antigens to bind to a single intact antibody which is subsequently detected as a single complex. This will reduce the sensitivity of the technique. The use of monovalent fragments eliminates this effect.

The Fc fragment of the antibody is associated with the activation of the complement system and rheumatoid factor (RF) binding. It is possible that intact antibodies attached to antigen in solution could be complexed by RF present in serum with the result that subsequent complex detection could be inhibited, e.g. by the RF binding interfering with binding to the solid phase support. The use of Fab type fragments (e.g. Fab, Fab' or $F(ab')_2$ which do not contain the Fc region) would avoid this effect.

In processes in which the labelled component reacts first with the antigen, the use of labelled fragment will result in a smaller complex, which will make binding to the solid phase support easier. The use of antibody fragments for the solid phase component will also make possible the production of a very high capacity solid phase—even higher than where monoclonal intact antibodies are used. The use of the smallest possible labelled fragments will also facilitate the use of more than one labelled entity since steric effects will be significantly reduced. The same considerations will hold when fragments are employed as reagent (c) in the procedures cited.

The following non-limiting examples are intended to illustrate the present invention.

Example 1

Assay of TSH

In this assay, selective binding of the reagent (c) to the solid phase reagent (d) was achieved by interaction of FITC conjugated and bound anti-FITC antibodies. Two $^{125}$I-labelled antibody reagents were employed.

Preparation of the starting materials
(i) Combined reagent comprising $^{125}$I-labelled monoclonal antibodies (two types) and FITC conjugated monoclonal antibodies.

Monoclonal antibodies were obtained from mouse ascites fluid by the process reported by Milstein and Köhler in Nature 256 495—497 (1975). Antibodies from individual hybridoma cell lines were screened to identify those producing antibody to discrete antigenic determinants. Those having the highest affinities to TSH were selected for use in the assay. Two types of antibodies were selected for labelling with $^{125}$I and one for conjungation with FITC. Each of those selected exhibited an affinity for TSH of greater than $1 \times 10^{10}$ litres/mole and did not interfere with the other's binding to TSH.

Monoclonal antibodies were labelled with $^{125}$I using the procedure of Hunter et al, J. Immunol. Methods 50, 133—144 (1982), and purified by gel filtration on Sephacryl® S-300, giving a product with an average of approximately 1 atom of $^{125}$I per antibody molecule.

Conjugation of FITC to monoclonal antibody was achieved by reacting 200 μg fluorescein isothiocyanate (FITC), Sigma London Chemical Co., England, with 5 mg antibody in 1.4 ml sodium bicarbonate buffer, 0.2 M, pH 9.0, for 18 hours at room temperature. The reaction mixture was purified by gel filtration on Sephadex® G-50 superfine, giving a product incorporating an average of 6 molecules FITC per antibody molecule.

The $^{125}$I-labelled monoclonal antibodies and FITC conjugated monoclonal antibody reagent were combined in a single reagent containing approximately $2 \times 10^6$ counts per minute per ml $^{125}$I-labelled antibodies and 2.5 μg/ml FITC conjugated antibody reagent in a buffer system of 0.05M sodium phosphate, pH 7.4 containing 0.5% (w/v) bovine serum albumin. 0.2% (v/v) normal (non-immune) sheep serum and 0.2% (w/v) sodium azide.

(ii) Solid phase reagent comprising anti-FITC polyclonal antibodies covalently linked to magnetisable cellulose particles

Anti-FITC was a conventional polyclonal antiserum obtained by immunising sheep with FITC conjugated to keyhole limpet haemocyanin. The magnetisable cellulose particles were a composite of cellulose containing approximately 50% black ferric(ous) oxide ($Fe_3O_4$), with mean particle diameter of 3 μm (see Forrest and Rattle, "Magnetic Particle Radio immunoassay", in Immunoassays for Clinical Chemistry, p. 147—162, Ed. Hunter and Corrie, Churchill Livingstone, Edinburgh (1983)). Anti-FITC antiserum was covalently coupled to the magnetisable cellulose following cyanogen bromide activation of the cellulose, according to the procedure of Axen *et al*, Nature *214*, 1302—1304 (1967). The antiserum was coupled at a ratio of 2 ml antiserum to 1 gram of magnetisable solid phase.

Anti-FITC magnetisable solid phase was diluted to a concentration of 6—8 mg/ml in sodium phosphate buffer 0.05M, pH 7.4 containing 0.25% (w/v) bovine serum albumin, 0.25% (v/v) Trion® X-100, 0.8% (w/v) hydroxypropyl methyl cellulose and 0.1% (w/v) sodium azide.

Determination of TSH

Solutions of TSH in normal human serum, calibrated against the 1st International Reference Preparation of human TSH for immunoassay (68/38), were used as standards.

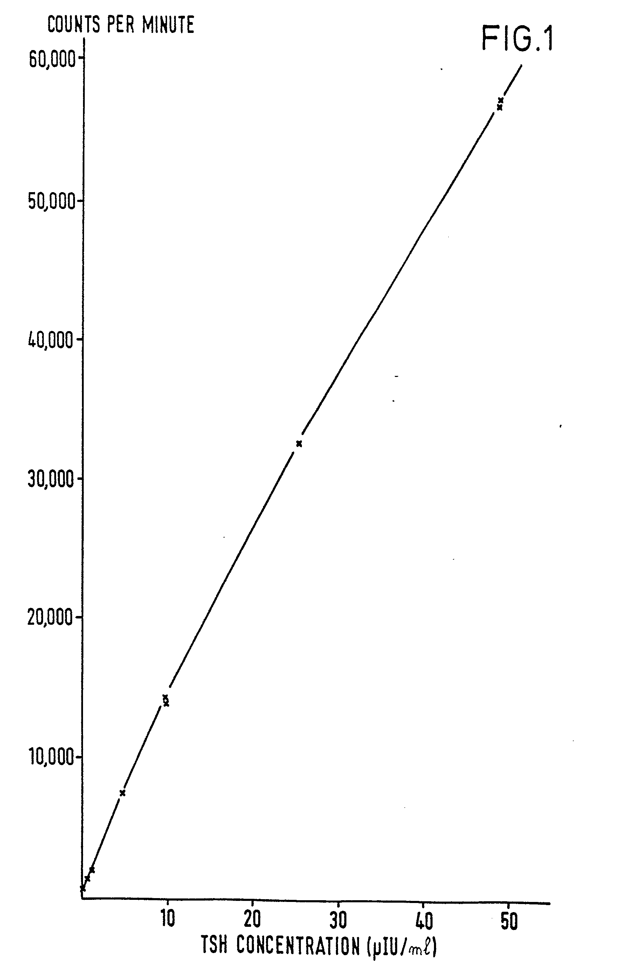

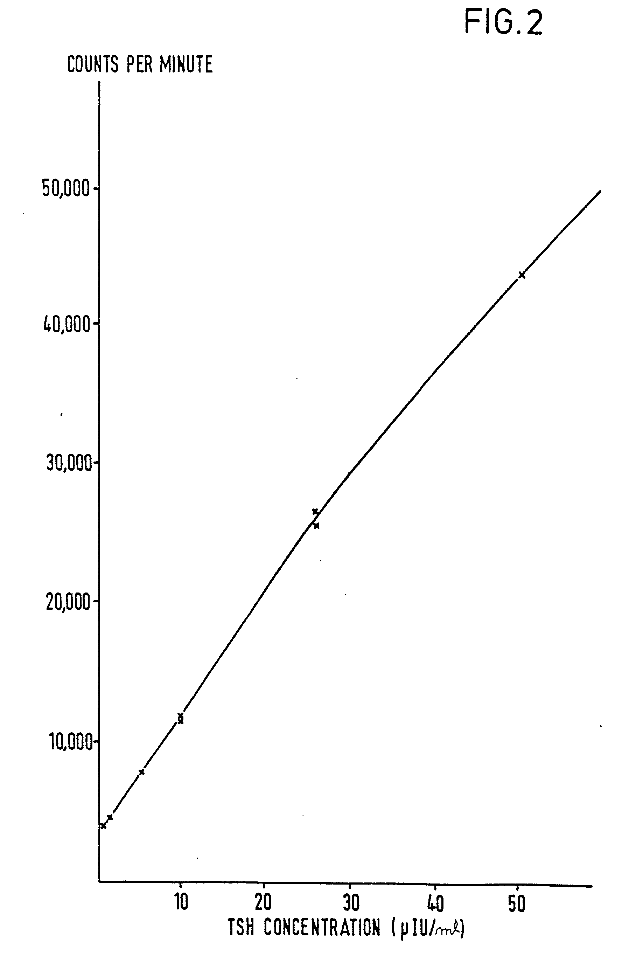

Duplicate samples were run in which 200 µl of specimen (serum) was added to 100 µl of the combined reagent containing [125]I-labelled antibodies and FITC conjugated antibody reagent and incubated for 2 hours at room temperature. 200 µl of anti-FITC magnetisable solid phase suspension was then added and incubated for 5 minutes, after mixing. The solid phase particles were separated magnetically, the liquid removed by decantation and the particles washed by addition of 1 ml buffer. Following this, the particles were again separated magnetically, the wash liquid removed by decantation and the resultant pellet counted for bound [125]I-labelled antibody. An example dose response curve is shown in Fig. 1. Counts per minute bound are plotted along the vertical axis and micro international units human TSH/ml are plotted along the horizontal axis.

Example 2
Assay of TSH

In this assay, selective binding of reagents (c) and (d) was carried out by the same method of Example 1, with Fab' fragments being used in place of intact [125]I-labelled antibodies.

Preparation of the starting materials
(i) Combined reagent comprising [125]I-labelled Fab' monoclonal antibody fragments and FITC conjugated monoclonal antibodies

F(ab')[2] fragments of mouse monoclonal anti-TSH were obtained by incubating 250 µg of thiol activated papain with 5 mg antibody in 1.5 ml of sodium acetate buffer 0.1M, pH 5.5 containing 0.003M EDTA for 18 hours at 37°C. The mixture was purified by chromatography on Sepharose® protein A followed by gel filtration on Sephacryl® S-200. Monovalent Fab' fragments were prepared by reacting 0.5 mg F(ab')[2] in a solution (0.6 ml) of β-mercaptoethylamine 0.1M in sodium phosphate buffer 0.1M pH 7.4 for 90 minutes at 20°C followed by purification by gel filtration on Sephacryl®S-200.

Fab' fragments were labelled with [125]I in an analogous manner to the intact monoclonal antibodies described in Example 1, giving a product with an average of greater than 1/10 atom [125]I per molecule of Fab'.

The [125]I-labelled Fab' anti-TSH was combined in a single reagent with FITC conjugated monoclonal antibody (prepared as described in Example 1), containing $2\times10^6$ counts per minute per ml [125]I-labelled Fab' and 2.5 µg/ml FITC conjugated antibody, in a buffer system of 0.05M sodium phosphate, pH 7.4 containing 0.5% (w/v) bovine serum albumin, 0.2% (w/v) normal (non-immune) sheep serum and 0.2% (w/v) sodium azide.

(ii) Solid phase reagent comprising anti-FITC polyclonal antibodies covalently linked to magnetisable cellulose particles.

Prepared in the manner described in Example 1.

Determination of TSH

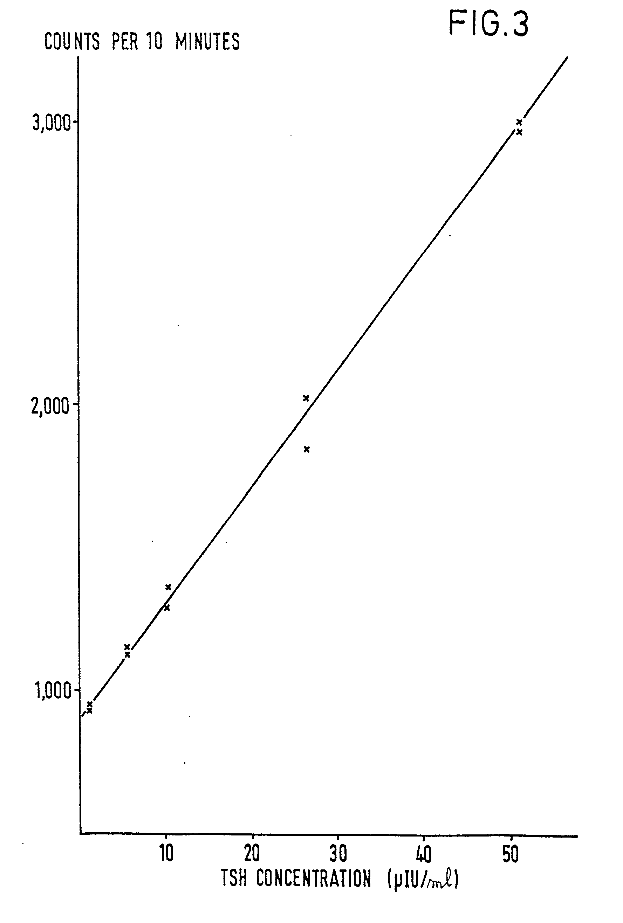

All other reagents, and the assay protocol for determination of TSH were as described in Example 1. An example dose response curve is shown in Fig. 3. Counts per ten minutes bound are plotted along the vertical axis and micro international units human TSH/ml are plotted along the horizontal axis.

**Claims**

1. A method of immonoassay of an antigen with more than one epitope in a liquid sample which comprises incubating a mixture of:
(a) the liquid sample,
(b) one or more populations of labelled antibodies to the antigen under assay,
(c) a reagent comprising antibodies to the antigen under assay conjugated with a reagent Z, and
(d) a reagent Y capable of binding to reagent Z by non-covalent bonding, but which is not directly bindable to either component (a) or component (b), the said reagent Y being mounted to a solid phase support and at least components (b) and (c) comprising monoclonal antibodies;
separating the solids fraction from the liquid fraction, determining the amount of label in one of the said fractions and, therefrom, the amount of antigen present in the sample.

2. A method as claimed in claim 1 wherein reagent Z is a hapten or antigenic substance and reagent Y comprises antibodies raised to the said reagent Z.

3. A method as claimed in claim 2 wherein reagent Z is a hapten selected from florescein isothiocyanate, rhodamine isothiocyanate, 2,4-dinitrofluorobenzene, phenyl isocyanate and dansyl chloride.

4. A method as claimed in any one of claims 1 to 3 wherein two populations of radioactively-labelled monoclonal antibodies to the antigen under assay are employed.

5. A method as claimed in any one of the preceding claims wherein at least one of the antibody reagents is in the form of fragments.

6. A kit of reagents for carrying out a method of immunoassay as claimed in any one of the preceding claims.

7. A kit of reagents as claimed in claim 6 comprising (i) particles or beads conjugated to the reagent Y, (ii) one or more populations of labelled monoclonal antibodies to the antigen under assay, (iii) a reagent comprising monoclonal antibodies to the antigen under assay conjugated with the reagent Z, and (iv) calibrated reference solutions.

8. A kit of reagents as claimed in claim 7 for carrying out a method as claimed in claim 4 for assaying thyroid stimulating hormone (TSH) comprising (i) magnetisable particles or beads conjugated to antifluorescein isothiocyanate (FITC) polyclonal antibodies, (ii) two populations of radioactively-labelled monoclonal antibodies to TSH (iii) a reagent comprising monoclonal antibodies to TSH conjugated with FITC and (iv) calibrated reference solutions.

## Patentansprüche

1. Immunoassayverfahren für ein Antigen mit mehr als einem Epitop in einer flüssigen Probe, wobei man eine Mischung inkubiert aus:

(a) der flüssigen Probe,

(b) einer oder mehreren Populationen von markierten Antikörpern gegen das zu untersuchende Antigen,

(c) einem Reagens, das Antikörper gegen das zu untersuchende Antigen, konjugiert mit einem Reagens Z, umfaßt und

(d) einem Reagens Y, das in der Lage ist, durch eine nicht-kovalente Bindung an das Reagens Z zu binden, das sich aber nicht direkt an die Komponente (a) oder die Komponente (b) binden läßt, wobei das Reagens Y an einen Festphasenträger gebunden ist und wenigstens die Komponenten (b) und (c) monoklonale Antikörper umfassen;

die Feststofffraktion von der Flüssigfraktion trennt, die Menge an markiertem Prudkt in einer der erwähnten Fraktionen und daraus die Menge an in der Probe vorhandenem Antigen bestimmt.

2. Verfahren nach Anspruch 1, wobei das Reagens Z ein Hapten oder eine antigene Substanz ist und das Reagens Y Antikörver gegen das Reagens Z umfaßt.

3. Verfahren nach Anspruch 2, wobei das Reagens Z ein Hapten ist, das ausgewält ist unter Fluoresceinisothiocyanat, Rhodaminisothiocyanat, 2,4-Dinitrofluorbenzol, Phenylisocyanat und Dansylchlorid.

4. Verfahren nach einem der Ansprüche 1 bis 3, wobei man zwei Populationen radioaktiv-markierter monoklonaler Antikörper gegen das zu untersuchende Antigen verwendet.

5. Verfahren nach einem der vorhergehenden Ansprüche, wobei wenigstens eines der Antikörperreagenzien in Form von Fragmenten vorliegt.

6. Reagenzien-Kit zur Durchführung des Immunoassayverfahrens nach einem der vorhergehenden Ansprüche.

7. Reagenzien-Kit nach Anspruch 6, umfassend (i) an das Reagens Y konjugierte Partikel oder Beads, (ii) eine oder mehrere Populationen markierter monoklonaler Antikörper gegen das zu untersuchende Antigen, (iii) ein Reagens, das monoklonale Antikörper gegen das zu untersuchende Antigen, konjugiert mit dem Reagens Z umfaßt und (iv) eingestellte Bezugslösungen.

8. Reagenzien-Kit nach Anspruch 7 zur Durchführung des Verfahrens nach Anspruch 4 zur Bestimmung des Thyreoidea-stimulierenden Hormons (TSH), umfassend (i) magnetisierbare Partikel oder Beads, die an Antifluoresceinisothiocyanat (FITC)-polyklonale Antikörper konjugiert sind, (ii) zwei Populationen radioaktiv markierter monoklonaler Antikörper gegen TSH, (iii) ein Reagens, das monoklonale Antikörper gegen TSH, konjugiert mit FITC umfaßt und (iv) eingestellte Bezugslösungen.

## Revendications

1. Procédé d'immunodosage d'un antigène avec plus d'un épitope dans un échantillon liquide, qui comprend la mise en incubation d'un mélange:

(a) de l'échantillon liquide,

(b) d'une ou plusieurs populations d'anticorps marqués contre l'antigène soumise au dosage,

(c) d'un réactif comprenant des anticorps contre l'antigène soumis au dosage conjugués avec un réactif Z, et

(d) d'un réactif Y capable de fixation sur le réactif Z par liaison non covalente, mais qui n'est pas susceptible de se lier directement au composant (a) ou au composant (b), ce réactif Y étant fixé sur un support en phase solide et au moins les composants (b) et (c) comprenant des anticorps monoclonaux;

la séparation entre la fraction des solides et la fraction de liquide, la détermination de la quantité de marqueur dans l'une de ces fractions et, à partir de celle-ci, de la quantité de l'antigène présent dans l'échantillon.

2. Procédé suivant la revendication 1, dans lequel le réactif Z est un haptène ou une substance antigénique et le réactif Y comprend des anticorps dirigés contre le réactif Z.

3. Procédé suivant la revendication 2, dans lequel le réactif Z est un haptène choisi entre l'isothiocyanate de fluoroescéine, l'isothiocyanate de rhodamine, le 2,4-dinitrofluorobenzène, l'isocyanate de phényle et le chlorure de dansyle.

4. Procédé suivant l'une quelconque des revendications 1 à 3, dans lequel deux populations d'anticorps monoclonaux marqués radioactivement contre l'antigène soumis au dosage sont utilisées.

5. Procédé suivant l'une quelconque des revendications précédentes, dans lequel au moins l'un des réactifs anticorps est sous la forme de fragments.

6. Trousse de réactifs pour l'exécution d'un procédé d'immunodosage suivant l'une quelconque des revendications précédentes.

7. Trousse de réactifs suivant la revendication 6, comprenant (i) des particules ou perles conjuguées au réactif Y, (ii) une ou plusieurs populations d'anticorps monoclonaux marqués contre l'antigène soumise au dosage (iii) un réactif comprenant des anticorps monoclonaux contre l'antigène soumis au dosage conjugués avec le réactif Z, et (iv) des solutions de référence étalonnées.

8. Trousse de réactifs suivant la revendication 7 pour l'exécution d'un procédé suivant la revendication 4 pour le dosage de l'hormone thyréotrope (TSH), comprenant (i) des particules ou perles magnétisables conjuguées avec des anticorps polyclonaux antiisothiocyanate de fluorescéine (FITC), (ii) deux populations d'anticorps monoclonaux marqués radioactivement contre TSH, (iii) un réactif comprenant des anticorps monoclonaux contre TSH conjugués avec FITC et (iv) des solutions de référence étalonnées.

FIG.1

FIG.2

0 105 714

2

0 105 714

FIG.3

COUNTS PER 10 MINUTES

TSH CONCENTRATION (µIU/ml)